(19) Europäisches Patentamt
European Patent Office
Office européen des brevets

(11) Veröffentlichungsnummer: **0 109 495**
**B1**

(12) # EUROPÄISCHE PATENTSCHRIFT

(45) Veröffentlichungstag der Patentschrift: **29.05.85**

(51) Int. Cl.⁴: **C 07 C 101/30**, C 07 C 99/00

(21) Anmeldenummer: **83108917.2**

(22) Anmeldetag: **09.09.83**

(54) **Verfahren zur Herstellung von beta-Hydroxy-alpha-aminocarbonsäuren.**

(30) Priorität: **19.11.82 DE 3242748**

(43) Veröffentlichungstag der Anmeldung: **30.05.84 Patentblatt 84/22**

(45) Bekanntmachung des Hinweises auf die Patenterteilung: **29.05.85 Patentblatt 85/22**

(84) Benannte Vertragsstaaten: **AT BE CH DE FR GB IT LI NL**

(56) Entgegenhaltungen:
**DE - A - 1 543 916**
**DE - A - 2 515 622**
**FR - A - 1 375 060**

**THE MERCK INDEX, 9. Auflage, 1976 MERCK & CO., INC., Rahway, N.J., U.S.A. ONR 15, Zeilen 2-13; ONR 85, Zeilen 28-31; ONR 86, Zeile 1**

(73) Patentinhaber: **Degussa Aktiengesellschaft, Weissfrauenstrasse 9, D-6000 Frankfurt am Main 1 (DE)**

(72) Erfinder: **Kleemann, Axel, Dr. Dipl.-Chem., Greifenhagenstrasse 25, D-6450 Hanau 9 (DE)**
Erfinder: **Lehmann, Bernd, Dr. Dipl.-Chem., Ringstrasse 24a, D-7750 Konstanz 19 (DE)**
Erfinder: **Deller, Klaus, Dr. Dipl.-Chem., Friedhofstrasse 47, D-6452 Hainburg (DE)**

EP 0 109 495 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der Erfindung ist ein Verfahren zur Herstellung von β-Hydroxy-α-aminocarbonsäuren der allgemeinen Formel

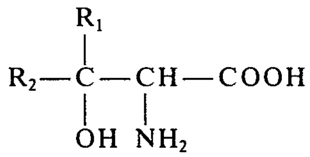

(I)

in der $R_1$ und $R_2$ gleich oder verschieden sind und jeweils Wasserstoff oder einen geradkettigen oder verzweigten Alkylrest mit 1 bis 10 Kohlenstoffatomen bedeuten, durch eine bekannte Aminosäuresynthese.

Aus der DE-OS 2 515 622 ist ein Verfahren zur Hestellung von β-Hydroxy-α-aminocarbonsäuren aus alkali-empfindlichen Vorläufern von β-Hydroxy-aldehyden mit Hilfe der gegebenenfalls modifizierten Strecker-Reaktion bekannt. Als alkali-empfindliche Vorläufer der β-Hydroxy-aldehyde dienen dabei unsubstituierte oder substituierte Vinylacetatepoxide, 2,5-Diacetoxydioxane, 2,5- Dichlordioxane, Monoacetoxyethylencarbonate, Monochlorethylencarbonate, Vinylencarbonate, Monochlorethylenoxide, Monoacetoxyacetaldehyde oder 2,2-Diacetoxy-ethanole. Diese Ausgangsmaterialien sind jedoch zumindest teilweise nur schwer zugänglich.

Das erfindungsgemäße Verfahren ist nun dadurch gekennzeichnet, daß man ein Cyanhydrin der allgemeinen Formel

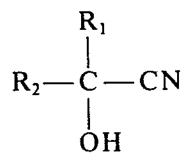

(II)

in der $R_1$ und $R_2$ die bereits genannte Bedeutung haben, in einem wasserhaltigen Medium, das pro Mol eingesetztem Cyanhydrin der allgemeinen Formel (II) mindstens 1 Mol Wasser enthält, in Gegenwart eines Palladium- oder Platinkatalysators und, bezogen auf das Cyanhydrin der allgemeinen Formel (II), eines Äquivalents einer anorganischen oder organischen Säure oder mindestens eines Äquivalents eines sauren Ionenaustauschers oder in Gegenwart von metallischem Nickel und, wiederum bezogen auf das Cyanhydrin der allgemeinen Formel (II), mindestens eines Äquivalents eines sauren Ionenaustauschers bei einer Temperatur zwischen $-20$ und $+25°$ C und einem Wasserstoffdruck von weniger als 10 bar hydriert, bis pro Mol eingesetztem Cyanhydrin der allgemeinen Formel (II) ein Mol Wasserstoff aufgenommen ist, den Katalysator und gegebenenfalls den Ionenaustauscher abtrennt und die verbleibende Lösung als Ausgangsmaterial für eine Aminosäuresynthese nach Strecker oder Bucherer einsetzt.

Die als Ausgangsmaterialien für das erfindungsgemäße Verfahren dienenden Cyanhydrine der allgemeinen Formel (II) können in einfachster Weise nach bekannten Methoden (vgl. z. B. Houben-Weyl: Methoden der organischen Chemie, 4. Auflage, Georg Thieme Verlag, Stuttgart, Band VIII, Seiten 274 bis 278) aus den entsprechenden Aldehyden oder Ketonen durch Umsetzung mit Blausäure hergestellt werden. Sofern die Herstellung der Cyanhydrine in wäßriger Lösung vorgenommen wird, können diese wäßrigen Lösungen unmittelbar für das erfindungsgemäße Verfahren eingesetzt werden.

Die zur Herstellung der Cyanhydrine der allgemeinen Formel (II) benötigten Aldehyde oder Ketone ihrerseits können erforderlichenfalls auch in an sich bekannter Weise (vgl. z. B. Houben-Weyl: Methoden der organischen Chemie, 4. Auflage, Georg Thieme Verlag, Stuttgart, Band VII/1, Seiten 13 bis 503, bzw. Band VII/2a-c) hergestellt werden.

Beispiele für nach dem erfindungsgemäßen Verfahren umzusetzende Cyanhydrine der allemeinen Formel (II) sind Propionaldehydcyanhydrin, Butyraldehydcyanhydrin, Isobutyraldehydcyanhydrin, Valeraldehydcyanhydrin, Hexanalcyanhydrin, Heptanalcyanhydrin, Octanalcyanhydrin, Butanoncyanhydrin, Pentanoncyanhydrin, Methylisopropylketoncyanhydrin oder Diisopropylketoncyanhydrin.

Besonders geeignet ist das erfindungsgemäße Verfahren für die Umsetzung von Glykolnitril zu D,L-Serin oder Milchsäurenitril zu D,L-Threonin.

Die Cyanhydrine der allgemeinen Formel (II) werden in einem wasserhaltigen Medium, das pro Mol eingesetztem Cyanhydrin mindestens 1 Mol Wasser enthalten muß, hydriert. Sofern die Löslichkeit des eingesetzten Cyanhydrins dies zuläßt, kann Wasser als alleiniges Lösungsmittel verwendet werden, sonst kommen auch Mischungen von Wasser mit wasserlöslichen Alkoholen, wie Methanol, Ethanol, n-Propanol, Isopropylalkohol, n-Butanol, Isobutylalkohol, sekundärer oder tertiärer Butylalkohol, Dioxan oder Tetrahydrofuran als Lösungsmittel in Frage. Das Lösungsmittel kann beispielsweise in einer Menge zwischen 0,5 und 100 ml, vorzugsweise zwischen 1 und 50 ml, pro Gramm eingesetztem

Cyanhydrin angewandt werden.

Die Hydrierung erfolgt entweder in Gegenwart eines Palladium- oder Platinkatalysators oder in Gegenwart von metallischem Nickel. Geeignete Katalysatoren sind beispielsweise metallisches Palladium, insbesondere als Palladiummohr, metallisches Platin, insbesondere als Platinschwarz, oder Platin-IV-oxid. Wird metallisches Palladium oder Platin verwendet, so kann es in freier Form eingesetzt werden, aber auch in Form von Trägerkatalysatoren mit beispielsweise Aktivkohle, Bariumsulfat, Aluminiumoxid oder Siliciumdioxid als Trägermaterial. Besonders bevorzugte Katalysatoren sind Palladiummohr oder Palladium auf Aktivkohle. Das metallische Nickel wird vorzugsweise in der aktivierten Form des Raney-Nickels eingesetzt. Es können auch Gemische von mehreren Katalysatoren eingesetzt werden. Die eingesetzte Menge an Katalysator ist nicht kritisch, zur Erzielung kurzer Reaktionszeiten empfiehlt es sich jedoch, den Katalysator, berechnet als aktives Metall, in einer Menge zwischen 0,1 und 100, vorzugsweise zwischen 1 und 10, Gewichtsprozent, bezogen auf das eingesetzte Cyanhydrin der allgemeinen Formel (II), anzuwenden.

Bei der Hydrierung ist ferner die Gegenwart einer Säure erforderlich. Bei Verwendung eines Palladium- oder Platinkatalysators kann dies eine organische Säure, wie Ameisensäure, Essigsäure oder Oxalsäure, sein. Bevorzugt werden jedoch Mineralsäuren, wie Schwefelsäure, Phosphorsäure und insbesondere Salzsäure, oder saure Ionenaustauscher verwendet. Die organischen oder anorganischen Säuren werden in der stöchiometrischen Menge von einem Äquivalent, bezogen auf das eingesetzte Cyanhydrin der allgemeinen Formel (II), zugesetzt. Die Anwendung eines Überschusses an Säure ist unzweckmäßig. Die sauren Ionenaustauscher müssen in einer Menge von mindestens einem Äquivalent, bezogen auf das eingesetzte Cyanhydrin der allgemeinen Formel (II), zugesetzt werden. In diesem Falle stört jedoch ein Überschuß nicht. Wird als Hydrierungskatalysator metallisches Nickel verwendet, wird als Säure ein saurer Ionenaustauscher in einer Menge von ebenfalls wieder mindestens einem Äquivalent, bezogen auf das eingesetzte Cyanhydrin der allgemeinen Formel (II), zugesetzt.

Die Hydrierung wird bei einer Temperatur zwischen —20 und +25° C, vorzugsweise zwischen 0 und 20° C, und bei einem Wasserstoffdruck von weniger als 10 bar, vorzugsweise zwischen 1 und 2 bar, durchgeführt. Besonders vorteilhaft ist es, wenn man einfach Wasserstoff durch das Reaktionsgemisch hindurchleitet. Nach Aufnahme von einem Mol Wasserstoff pro Mol eingesetztem Cyanhydrin der allgemeinen Formel (II) wird die Hydrierung beendet.

Nun wird der Katalysator und gegebenenfalls der Ionenaustauscher beispielsweise durch Abfiltrieren oder Zentrifugieren, abgetrennt. Die jeweilige genaue Zusammensetzung der verbleibenden Lösung ist nicht im einzelnen bekannt, sie verhält sich jedoch wie eine Lösung eines $\alpha$-Hydroxyaldehyds der allgemeinen Formel

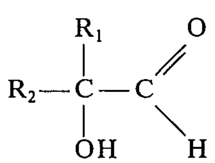

(III)

in der $R_1$ und $R_2$ die bereits genannte Bedeutung haben. So kann z. B. mittels Hydroxylamin-hydrochlorid das entsprechende Oxim in nahezu theoretischer Menge, bezogen auf das eingesetzte Cyanhydrin der allgemeinen Formel (II), erhalten werden.

Diese Lösung kann daher unmittelbar als Ausgangsmaterial für eine Aminosäuresynthese nach Strecker oder Bucherer eingesetzt werden. Zur Synthese nach Strecker wird die Lösung mit Blausäure oder einem Salz derselben und mit Ammoniak oder einem Ammoniumsalz zu dem entsprechenden $\beta$-Hydroxy-$\alpha$-aminonitril umgesetzt und dieses anschließend sauer oder basisch verseift. Zur Synthese nach Bucherer wird die Lösung mit Blausäure oder einem Salz derselben und mit Ammoniak und Kohlendioxid oder mit Ammoniumcarbonat zur Reaktion gebracht und das hierbei gebildete, in 5-Stellung substituierte Hydantoin anschließend basisch verseift. Aus der jeweiligen Verseifungslösung kann dann die gebildete $\beta$-Hydroxy-$\alpha$-aminocarbonsäure in bekanner Weise, z. B. mit Hilfe eines sauren Ionenaustauschers, gewonnen werden.

Die Erfindung wird durch die nachfolgenden Beispiele näher erläutert. Prozentangaben bedeuten, sofern nicht anders angegeben, Gewichtsprozente.

Beispiel 1

103,6 g (1 Mol) 55%iges wäßriges Glykolnitril werden unter Kühlung mit 197 g (1 Mol) 18,5%iger Salzsäure versetzt. Nach Zugabe von 1,14 g Pd auf Aktivkohle (10%ig) wird bei 6 bar Wasserstoffdruck unter Rühren bei 25° C bis zur Aufnahme von 1 Mol Wasserstoff hydriert. Dann wird der Katalysator abfiltriert.

Das Filtrat wird bei 20 bis 25° C in eine Lösung von 51,2 g (1,04 Mol) Natriumcyanid und 64 g Ammoniak in 210 ml Wasser eindosiert.

Man erwärmt auf 35° C und rührt 3 Stunden bei dieser Temperatur. Anschließend wird der Ammoniaküberschuß im Vakuum abgezogen. Der Rückstand wird mit 493 g (5 Mol) konzentrierter Salzsäure versetzt und 4 Stunden zum Sieden erhitzt.

Durch Ionenaustauschchromatographie in einem Aminosäureanalysator wird eine Serinausbeute von 82,2 g (78% der Theorie) quantitativ bestimmt.

Das D,L-Serin läßt sich z. B. mit Hilfe eines sauren Ionenaustauschers isolieren.

## Beispiel 2

Man verfährt wie im Beispiel 1 mit dem Unterschied, daß anstelle von Pd auf Aktivkohle 1,14 g Pd-Mohr eingesetzt werden.

Die Ausbeute an Serin beträgt 84 g (80% der Theorie).

## Beispiel 3

Man verfährt wie im Beispiel 2, führt die Hydrierung jedoch bei Normaldruck durch.

Die Ausbeute an Serin beträgt 82 g (78% der Theorie).

## Beispiel 4

Man verfährt wie im Beispiel 1 mit dem Unterschied, daß anstelle von Pd auf Aktivkohle 1,14 g metallisches Platin eingesetzt werden.

Die Ausbeute an Serin beträgt 79,5 g (76% der Theorie).

## Beispiel 5

Man verfährt wie im Beispiel 1 mit dem Unterschied, daß anstelle von Palladium auf Aktivkohle 1,14 g Pd auf $BaSO_4$ (10%ig) eingesetzt werden.

Die Ausbeute an Serin beträgt 83 g (79% der Theorie).

## Beispiel 6

Man verfährt wie im Beispiel 1 mit dem Unterschied, daß anstelle von Glykolnitril eine Lösung von 71 g (1 Mol) Milchsäurenitril in 58 g Wasser eingesetzt wird. Durch Ionenaustauschchromatographie in einem Aminosäureanalysator wird eine Threoninausbeute von 83 g (70% der Theorie) quantitativ bestimmt.

## Beispiel 7

Man verfährt wie im Beispiel 1 mit dem Unterschied, daß anstelle von Glykolnitril eine Lösung von 85 g Acetoncyanhydrin in 80 g Wasser eingesetzt wird.

Es wird eine Ausbeute von 90 g (68% der Theorie) an $\beta$-Hydroxyvalin bestimmt.

## Beispiel 8

18,7 g (0,18 Mol) 55%iges wäßriges Glykolnitril und 90 ml Wasser werden mit 30 ml eines sauren Ionenaustauschers (Lewatit S 100) und 1,2 g Pd auf Aktivkohle (10%ig) versetzt und bei 25°C unter Normaldruck mit Wasserstoffgas bis zur Aufnahme von 0,18 Mol Wasserstoff hydriert. Nach dem Abfiltrieren des Katalysators und Ionenaustauschers wird die erhaltene Lösung im Vakuum auf die Hälfte eingeengt. Dieses Konzentrat wird in eine Lösung von 9,3 g (0,19 Mol) Natriumcyanid, 10,2 g Ammoniumchlorid und 12 g Ammoniak in 40 ml Wasser eindosiert. Man erwärmt auf 35°C und rührt 3 Stunden bei dieser Temperatur. Anschließend wird der Ammoniaküberschuß im Vakuum abgezogen. Der Rückstand wird mit 94 g (0,9 Mol) konzentrierter Salzsäure versetzt und 4 Stunden zum Sieden erhitzt.

Die Ausbeute an D,L-Serin beträgt 14,3 g (76% der Theorie).

Beispiel 9

Man verfährt wie in Beispiel 8, setzt jedoch statt Palladium auf Aktivkohle 2,2 g Raney-Nickel als Katalysator ein.

Die Ausbeute an D,L-Serin beträgt 14,1 g (75% der Theorie).

Beispiel 10

33,3 g (0,3 Mol) 51,4%iges wäßriges Glykolnitril werden unter Kühlung mit 0,3 Äquivalenten Säure laut Tabelle 1 und 225 ml $H_2O$ vesetzt. Nach Zugabe von 2,5 g Pd auf Aktivkohle (10%ig) wird bei 2,5 bar Wasserstoffdruck unter Rühren bei 25°C bis zur Aufnahme von 0,3 Mol Wasserstoff hydriert. Dann wird der Katalysator abfiltriert.

Das Filtrat wird bei 20 bis 25°C in eine Lösung von 15,4 g (0,31 Mol) Natriumcyanid und 19,2 g Ammoniak in 63 ml Wasser eindosiert.

Man erwärmt auf 35°C und rührt 3 Stunden bei dieser Temperatur. Anschließend wird der Ammoniaküberschuß im Vakuum abgezogen. Der Rückstand wird mit 148 g (1,5 Mol) konzentrierter Salzsäure versetzt und 4 Stunden zum Sieden erhitzt.

Durch Ionenaustauschchromatographie in einem Aminosäureanalysator wird die Serinausbeute laut Tabelle 1 bestimmt.

Tabelle 1

| Versuch | Säure | Serinausbeute |
|---|---|---|
| a | Schwefelsäure | 23,8 g (75% der Theorie) |
| b | Phosphorsäure | 22,8 g (72% der Theorie) |
| c | Ameisensäure | 16,5 g (52% der Theorie) |
| d | Essigsäure | 15,2 g (48% der Theorie) |
| e | Oxalsäure | 20,0 g (63% der Theorie) |

Beispiel 11

33,3 g (0,3 Mol) 51,4%iges wäßriges Glykolnitril werden unter Kühlung mit 30 g Salzsäure — 37%ig (0,3 Mol) und 200 ml einer Mischung aus Wasser und einem wasserlöslichen Alkohol, Dioxan bzw. Tetrahydrofuran laut Tabelle 2 versetzt. Nach Zugabe von 2,5 g Pd auf Aktivkohle (10%ig) wird bei 2,5 bar Wasserstoffdruck unter Rühren bei 25°C bis zur Aufnahme von 0,3 Mol Wasserstoff hydriert. Dann wird der Katalysator abfiltriert.

Das Filtrat wird bei 20 bis 25°C in eine Lösung von 15,4 g (0,31 Mol) Natriumcyanid und 19,2 g Ammoniak in 63 ml Waser eindosiert.

Man erwärmt auf 35°C und rührt 3 Stunden bei dieser Temperatur. Anschließend wird der Ammoniaküberschuß im Vakuum abgezogen. Der Rückstand wird mit 148 g (1,5 Mol) konzentrierter Salzsäure versetzt und 4 Stunden zum Sieden erhitzt.

Durch Ionenaustauschchromatographie in einem Aminosäureanalysator wird die Serinausbeute laut Tabelle 2 bestimmt.

Tabelle 2

| Versuch | Lösungsmittel | Serinausbeute |
|---|---|---|
| a | 100 ml $H_2O$ + 100 ml Ethanol | 24,7 g (≙ 78% der Theorie) |
| b | 40 ml $H_2O$ + 160 ml Ethanol | 14,9 g (≙ 47% der Theorie) |
| c | 20 ml $H_2O$ + 180 ml Ethanol | 12,7 g (≙ 40% der Theorie) |
| d | 180 ml $H_2O$ + 20 ml Ethanol | 21,5 g (≙ 68% der Theorie) |
| e | 100 ml $H_2O$ + 100 ml Dioxan | 19,6 g (≙ 62% der Theorie) |
| f | 180 ml $H_2O$ + 20 ml Dioxan | 20,3 g (≙ 64% der Theorie) |
| g | 100 ml $H_2O$ + 100 ml Tetrahydrofuran | 20,3 g (≙ 64% der Theorie) |
| h | 180 ml $H_2O$ + 20 ml Tetrahydrofuran | 17,4 g (≙ 55% der Theorie) |
| i | 100 ml $H_2O$ + 100 ml iso-Propanol | 22,2 g (≙ 70% der Theorie) |
| k | 100 ml $H_2O$ + 100 ml n-Butanol | 22,2 g (≙ 70% der Theorie) |

**Patentansprüche**

1. Verfahren zur Herstellung von $\beta$-Hydroxy-$\alpha$-aminocarbonsäuren der allgemeinen Formel

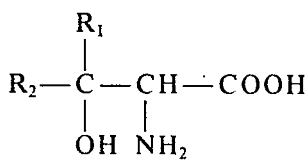

(I)

in der $R_1$ und $R_2$ gleich oder verschieden sind und jeweils Wasserstoff oder einen geradkettigen oder verzweigten Alkylrest mit 1 bis 10 Kohlenstoffatomen bedeuten, durch eine bekannte Aminosäuresynthese, dadurch gekennzeichnet, daß man ein Cyanhydrin der allgemeinen Formel

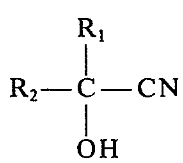

(II)

in der $R_1$ und $R_2$ die bereits genannte Bedeutung haben, in einem wasserhaltigen Medium, das pro Mol eingesetztem Cyanhydrin der allgemeinen Formel (II) mindestens 1 Mol Wasser enthält, in Gegenwart eines Palladium- oder Platinkatalysators und, bezogen auf das Cyanhydrin der allgemeinen Formel (II), eines Äquivalents einer anorganischen oder organischen Säure oder mindestens eines Äquivalents eines sauren Ionenaustauschers oder in Gegenwart von metallischem Nickel und, wiederum bezogen auf das Cyanhydrin der allgemeinen Formel (II), mindestens eines Äquivalents eines sauren Ionenaustauschers bei einer Temperatur zwischen −20 und +25° C und einem Wasserstoffdruck von weniger als 10 bar hydriert, bis pro Mol eingesetztem Cyanhydrin der allgemeinen Formel (II) ein Mol Wasserstoff aufgenommen ist, den Katalysator und gegebenenfalls den Ionenaustauscher abtrennt und die verbleibende Lösung als Ausgangsmaterial für eine Aminosäuresynthese nach Strecker oder Bucherer einsetzt.

2. Verfahren nach Anspruch 1, dadurch gekennzeichnet, daß man die Hydrierung in Gegenwart eines Palladium- oder Platinkatalysators und von Salzsäure durchführt.

3. Verfahren nach Anspruch 1 oder 2, dadurch gekennzeichnet, daß man die Hydrierung bei einer Temperatur zwischen 0 und 20° C durchführt.

4. Verfahren nach einem der Ansprüche 1 bis 3, dadurch gekennzeichnet, daß man die Hydrierung bei einem Wasserstoffdruck zwischen 1 und 2 bar durchführt.

5. Verfahren nach einem der Ansprüche 1 bis 4, dadurch gekennzeichnet, daß man die Hydrierung in Wasser oder in einem Gemisch aus Wasser und einem wasserlöslichen Alkohol, Dioxan oder Tetrahydrofuran durchführt.

**Claims**

1. A process for the production of $\beta$-hydroxy-$\alpha$-amino-carboxylic acids corresponding to the general formula

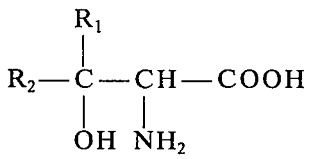

(I)

wherein, $R_1$ and $R_2$ are the same or different and in each case represent hydrogen or a straight chain or branched alkyl radical having from 1 to 10 carbon atoms, by a known amino acid synthesis, characterised in that a cyanohydrin of the general formula

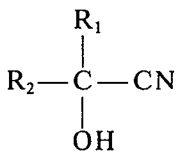

(II)

wherein, $R_1$ and $R_2$ are as defined above, is hydrogenated in an aqueous medium, which contains at least one mol of water per mol of cyanohydrin of general formula (II) used, in the presence of a palladium or a platinum catalyst and, based on the cyanohydrin of general formula (II), one equivalent of an inorganic or organic acid or at least one equivalent of an acidic ion exchanger or in the presence of metallic nickel and, again based on the cyanohydrin of general formula (II), at least one equivalent of an acidic ion exchanger, at a temperature of from −20 to +25° C and a hydrogen pressure of less than 10 bars, until 1 mol of hydrogen is absorbed per mol of cyanohydrin of general formula (II) used, the catalyst and optionally the ion exchanger are separated and the remaining solution is used as a starting material for an amino acid synthesis according to Strecker or Bucherer.

2. A process according to claim 1, characterised in that the hydrogenation is carried out in the presence of a palladium or a platinum catalyst and hydrochloric acid.

3. A process according to claim 1 or 2, characterised in that the hydrogenation is carried out at a temperature of from 0 to 20° C.

4. A process according to one of claims 1 to 3, characterised in that the hydrogenation is carried out at a hydrogen pressure of from 1 to 2 bars.

5. A process according to one of claims 1 to 4, characterised in that the hydrogenation is carried out in water or in a mixture of water and a water miscible alcohol, dioxan or tetrahydrofuran.

**Revendications**

1. Procédé pour la fabrication d'acides $\beta$-hydroxy-$\alpha$-aminocarboxyliques répondant à la formule générale:

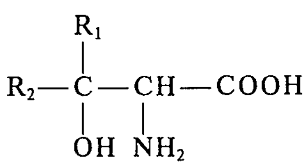

(I)

où $R_1$ et $R_2$ sont semblables ou différents, et représentent chaque fois un hydrogène ou un reste alcoyle à chaîne droite ou ramifiée, à 1 à 10 atomes de carbone, par une synthèse connue des acides aminés, procédé caractérisé en ce que l'on hydrogène une cyanhydrine de formule générale:

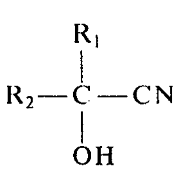

(II)

où $R_1$ et $R_2$ ont la signification déjà indiquée, dans un milieu contenant de l'eau, la cyanhydrine de la formule générale (II) mise en oeuvre contenant, par mole, au moins une mole d'eau, en présence d'un catalyseur au palladium ou au platine, et, calculé sur la cyanhydrine de formule générale (II), d'un équivalent d'un acide minéral ou organique ou d'au moins un équivalent d'un échangeur d'ions acide,

ou en présence de nickel métal, et, calculé à nouveau sur la cyanhydrine de formule générale (II), au moins un équivalent d'un échangeur d'ions acide, à une température qui se situe entre −20 et +25° C, et sous une pression d'hydrogène inférieure à 10 bars, jusqu'à ce qu'il soit absorbé, par mole de cyanhydrine de formule générale (II) mise en oeuvre, une mole d'hydrogène, sépare le catalyseur et éventuellement l'échangeur d'ions, et utilise la solution restante comme matière de départ pour une synthèse d'acides aminés suivant Strecker ou Bucherer.

2. Procédé suivant la revendication 1, caractérisé en ce que l'on exécute l'hydrogénation en présence d'un catalyseur au palladium ou au platine et d'acide chlorhydrique.

3. Procédé suivant les revendications 1 ou 2, caractérisé en ce qu'on exécute l'hydrogénation à une température qui se situe entre 0 et 20° C.

4. Procédé suivant l'une des revendications 1 à 3, caractérisé en ce que l'on exécute l'hydrogénation sous une pression d'hydrogène qui se situe entre 1 et 2 bars.

5. Procédé suivant l'une des revendications 1 à 4, caractérisé en ce que l'on exécute l'hydrogénation dans l'eau ou dans un mélange d'eau et d'un alcool soluble à l'eau, de dioxane ou de tétrahydrofuranne.